# EUROPEAN PATENT APPLICATION

(11) **EP 2 653 172 A2**
(43) Date of publication of application: **23.10.2013**
(21) Application number: 13164785.1
(22) Date of filing: 22.04.2013
(51) Int. Cl.: A61L 9/22, B01D 53/32, F24F 3/16

(54) **Air conditioning apparatus and ion generator device**

(30) Priority: 20.04.2012 IT PD20120125
(71) Applicant: Laboratori Archa S.r.l., 56121 Pisa (IT); Cliber S.r.l., 37044 Cologna Veneta (VR) (IT)
(72) Inventor: VALENTINI, Giorgio, I-56124 PISA (IT); TAMAI, Enrico, I-30030 Maerne di Martellago (VE) (IT)
(74) Representative: Gallo, Luca

(57) **Abstract**

Equipment for controlling contaminants in a gas flow, which comprises a main duct (2) for transporting a main flow (F) of contaminated gas and a by-pass circuit (4) equipped with an inlet opening (5) and an outlet opening (6) in connection with the main duct (2) for the extraction and the re-introduction of a secondary gas flow (Fb) from/into the main duct (2) respectively through the inlet opening (5) and the outlet opening (6). The outlet opening (6) is arranged downstream of the inlet opening (5) with respect to the direction of advancement (X) of the main flow (F) of contaminated gas in the main duct. The equipment further comprises circulation means (7) for making the secondary gas (F_{b}) flow from the inlet opening (5) to the outlet opening (6), means for generating oxidising chemical species (3) arranged to intercept the by-pass circuit (4) and adapted to generate in the secondary flow (F_{b}) the oxidising chemical species in an excess amount with respect to the amount required to oxidise the contaminant chemical species contained in the secondary flow (F_{b}), a first base amount of the oxidising chemical species being adapted to react with the contaminant chemical species contained in the secondary flow (F_{b}) and a second excess amount of the oxidising chemical species being adapted to be transported by the secondary flow (F_{b}) in the main flow (F). First mixing means (8) are arranged in the main duct (2) downstream of the outlet opening (6) in order to mix the secondary flow (F_{b}), containing the second excess amount of the oxidising chemical species, with the main flow (F).

## Description

### Field of application

The present invention regards an equipment and a process for controlling contaminants in a gas flow, according to the preamble of the respective independent claims. The equipment and the process subject of this document are intended to be advantageously used for controlling the contaminant and/or odoriferous substances, present within the cold gaseous emissions, and in particular for reducing the VOCs (*Volatile Organic Compounds*), VICs (*Volatile Inorganic Compounds*) and odoriferous compounds present within a gas flow for example intended to be conveyed into the atmosphere.

More in detail, the equipment and the process according to the present invention are applied in various fields, such as for example the chemical, petrochemical, leather processing and metalwork industry sectors for the treatment of gases developing during industrial processes, the automotive industry for the treatment of vehicle exhaust gases, the catering and baking industry for the reduction of fumes and odours developed in kitchens and ovens, farming and zootechnics industry in particular for the reduction of odours deriving from animal farms, the waste management industry, in particular in the treatment of air deriving from composting processes, or the civil and industrial waste purification sector, in particular for the depollution treatment and the removal of odours from the emissions produced by the basins for sludge purification and treatment.

### Prior art

Both at industrial and civil level over the years there has been felt the problem of reducing the contaminant and/or odoriferous substances present in the gaseous emissions released into the atmosphere, with the aim of reducing the environmental impact due to such emissions and with the aim or reducing the odour discomfort caused thereby to people occupying the areas affected by the presence of such emissions.

For such purposes, over the last decades there have been provided various equipment adapted to act on the gaseous emissions to reduce the concentration of contaminant and/or odoriferous compounds present therein. Conventionally such apparatus are based on two main operating principles, i.e. the separation of the contaminants from the gaseous flow, for example through transfer of matter by means of absorption processes (scrubber), adsorption, conventional filtration or by electrostatic means, and the transformation of the contaminants in chemical species characterised by less harmful effects on the environment and people, for example by means of oxidation processes, that may utilise supplementary fuel (combustion or thermo-destruction) or simple oxidants, such as air or oxygen, possibly assisted by the presence of heterogeneous catalysts.

Generally, the oxidation processes tend to completely transform the VOCs into water and carbon dioxide, having a minimum environmental impact and absence of odours, and the transformation of VICs possibly present in the flows of treated gases, among which the most common compounds are carbon monoxide, nitrogen and sulphur oxides, ammonium and hydrogen sulphide, into maximum oxidation compounds, which may be removed afterwards.

However, the oxidation processes, same case applying to all chemical transformation processes, are characterised by their own efficiency and, generally, they are not capable of affecting all the contaminant chemical species present and, often, they do not occur in a manner such to produce complete oxidation. The gaseous emissions treated by means of equipment in which oxidation processes take place, thus, besides the complete oxidation products, generally also comprise substances deriving from the partial oxidation of the contaminant chemical species present in the gaseous emissions before the treatment thereof.

Therefore, should the oxidation processes reveal poor efficiency, in the treated gaseous emissions there remain considerable concentrations of partial oxidation products such as, for example, alcohols, ketones, aldehydes or organic acids which, besides constituting contaminant substances, are characterised by a high hydrogen impact and may cause a considerable odour discomfort to the occupants of the areas affected by the aforementioned emissions.

Therefore, over the last years there have been various efforts aimed at developing technologies capable of obtaining oxidation transformations, as much as possible, of the substances responsible for the atmospheric contamination and pollution and, specifically, the substances that also produce unwanted odour effects.

In addition, there has been a growing interest towards the use Non Thermal Plasma (NTP) generator devices, i.e. a plasma produced by electrical discharges into the air at ambient temperature and pressure. The reactive species present in this type of plasma (atomic oxygen, OH radicals, ozone, superoxide etc.) actually have a high oxidant power not only towards a large variety of contaminant and/or pollutant compounds including the ones resistant to other types of oxidation treatments, but also towards biological microorganisms, such as for example bacteria and spores. Non thermal plasma technologies reveal various advantages, in particular, low installation and maintenance costs, high energy efficiency especially as regards the reduction of pollutants present in low concentration in the gaseous flow to be treated and, especially, a large spectrum of applicability.

Thus, over the last few years, there have been provided equipment for reducing volatile organic compounds and removing odoriferous compounds present in gaseous flows, which are provided with non thermal plasma generator devices.

For example patent US6991771 discloses an equipment and a process of this known type for the reduction of nitrogen oxides, quicksilver and sulphur dioxide present in a gas flow. In particular, the equipment described therein comprises a non thermal plasma generator device, preferably of the dielectric barrier discharge (DBD) type, an absorption device (scrubber) and a wet electro-static precipitator (WESP). The process comprises a first step for oxidising at least one part of the nitrogen oxides into nitric acid and at least one part of sulphur dioxide into sulphuric acid, a second step for absorbing at least one part of the nitrogen acids and sulphur dioxide by means of an ammonium-based absorption solution and a step for reducing the nitrogenised fumes, containing quicksilver oxide by means of the electrostatic precipitator.

However, the equipment and process briefly described above revealed some drawbacks in practice. In particular, the process is particularly complex to implement and the equipment required for the implementation thereof is characterised by a considerable overall dimension even if it processes low flow rates of gases. This leads to high costs of manufacture and maintenance of the equipment, as well as high costs for operating the equipment to implement the process described above.

Furthermore, there are known methods for controlling contaminants in a gas flow which provide for the insufflation into a main duct, within which there flows a contaminated gas flow to be treated, an auxiliary gas flow containing oxidising chemical species, which is generated outside the main duct. Such methods allow reducing the concentration of contaminant chemical species present in the treated gas flow given that they produce a partial oxidation of the contaminants by means of the oxidising chemical species introduced into the main duct with the auxiliary gas flow and in that they cause a dilution of the contaminated gas flow with an uncontaminated flow. The efficiency of reducing the contaminant chemical species by means of such process is quite low. The concentration of the contaminant chemical species present in the treated gas flow actually mainly reduces due to dilution, while actually the amount of contaminant chemical species carried by the treated gas flow and possibly introduced into the atmosphere is solely partly reduced due to oxidation.

Document EP 1980317 discloses an equipment for controlling contaminants in a gas flow, comprising a main duct for transporting a main flow of contaminated gas, which is intercepted by an air purification unit. The equipment further comprises a secondary circuit, which draws a portion of the main flow from a section downstream of the air purification unit before reintroducing it into a section upstream of the air purification unit after injecting ionised charges into it produced by a special ionisation unit intercepting the aforementioned secondary flow. Such air purification unit is arranged on the side upstream of a blowing fan and it is provided with a photocatalytic filter equipped with an electrostatic filter and a filter support made of titanium apatite. The photo-catalytic function of titanium apatite is activated by the ionised charges provided by the ionisation unit. Thus, the air purification unit is advantageously forced to treat not only the entire flow but also the recirculation portion in the secondary duct.

Document EP 1348448 discloses an equipment for controlling contaminants in a gas flow, comprising a main duct for transporting a main flow of contaminated gas subjected to a filtering action from which there departs a secondary duct parallel to the main one, which draws a portion of the main flow and treats it by means of an ion generation unit.

The air which enters into the secondary duct and which has acquired ions from the ion generator is blown through a secondary outlet into the environment diverting it towards the outlet of the main duct so as to join the two air flows. The positive and negative ions are carried by the airflow introduced by the main air intake for spreading in the entire environment and react with the contaminating species dispersed in the environment.

### Disclosure of the invention

In this framework the problem on which the present invention is based is that of overcoming the problem of the aforementioned prior art, by providing an equipment for controlling contaminants in a gas flow, which allows reducing, in an extremely efficient manner, the contaminant, pollutant and/or odoriferous substances present in a gas flow.

Another object of the present invention is that of providing an equipment for controlling contaminants in a gas flow, capable of allowing treating high volumes of gas with low overall dimensions.

Another object of the present invention is to provide an equipment for controlling contaminants in a gas flow, that is simple and inexpensive to manufacture and entirely reliable from an operative point of view.

Another object of the present invention is to provide an equipment, that is capable of maintaining high performance over time, even in the long-term, and capable of guaranteeing long-lasting efficiency at reducing contaminant, pollutant and/or odoriferous substances present in a gaseous flow even upon the variation of the chemical characteristics of the gaseous flow.

Another object of the present invention is to provide an equipment that is capable of continuously controlling the contaminated emissions, but which is also capable of being activated automatically only if the gaseous flow requires a purification treatment.

A further object of the present invention is to provide a process for controlling contaminants in a gas flow, that is simple and efficient.

### Brief description of the drawings

The technical characteristics of the invention, according to the aforementioned objects, are clearly observable from the contents of the claims indicated below and the advantages thereof shall be clearer in the following detailed description, provided with reference to the attached drawings, which represent an embodiment thereof purely by way of non-limiting example, wherein:

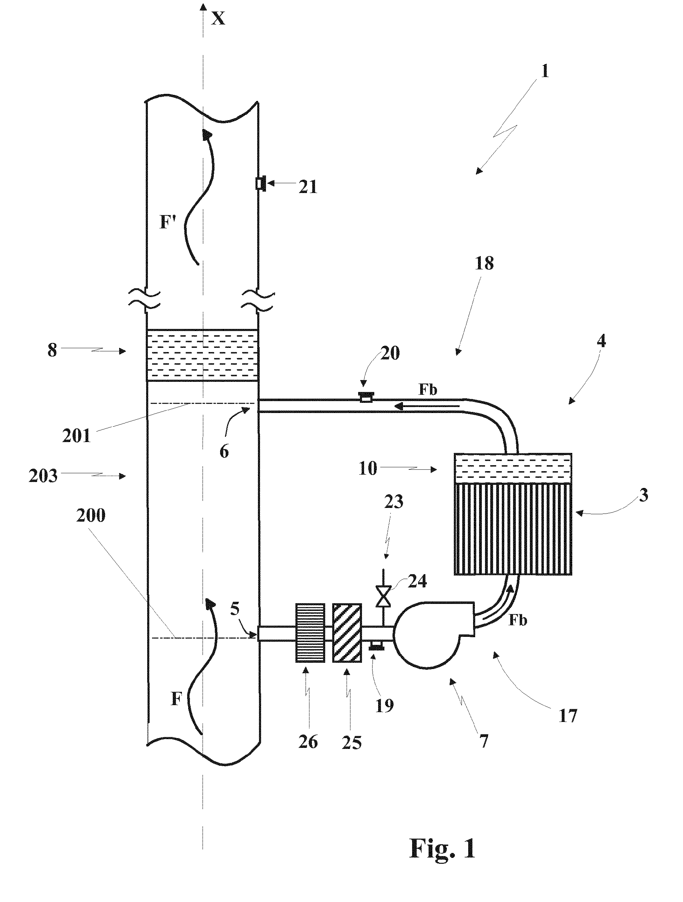

**FIG. 1** shows a schematic view of an equipment for controlling contaminants in a gas flow according to the present invention;
**FIG. 2** shows a schematic view of an equipment for controlling contaminants in a gas flow according to the present invention, during the use thereof in a method for calibrating the operating parameters of the equipment;
**FIG. 3** shows a sectional schematic view of a detail of the equipment according to the present invention, regarding a group of capacitors housed in respective containment pipes;
**FIG. 4** shows a front schematic view of the group of capacitors of Fig. 3;
**FIG. 5** shows a sectional view of a containment tube of a capacitor.

### Detailed description of a preferred embodiment

With reference to the attached drawings an equipment for controlling contaminants in a gas flow, subject of the present invention is indicated in its entirety with 1.

The equipment 1 and the process according to the invention are intended to be used in many and various sectors, both at industrial and civil level, with the aim of producing emissions with a lower environmental impact and/or that do not cause odour discomfort.

In particular, the equipment 1 and the process according to the present invention may be used for reducing the concentration of organic and/or inorganic and/or odoriferous substances in vapour state present in gaseous flows for example produced in chemical, petrochemical, leather processing, metalwork, automotive, wood or food processing industries, in the industrial processes for the production of paint and varnish or for the production of plastic material, in the industrial and/or civil waste purification industry, in waste disposal and treatment plants, in vehicle body building sites and in paint manufacturing plants, as well as in the agricultural and zootechnics industry, for example in animal farms and composting plants.

The equipment 1 according to the present invention may be used alone or combined with other equipment for the treatment of gaseous emissions arranged upstream and/or downstream of the equipment 1, without departing from the scope of protection defined by the present patent.

The equipment 1 was mainly studied so as to be used for controlling contaminants in cold gaseous flows, i.e. in gaseous flows having a temperature typically lower than 60°C and, in particular, preferably comprised between - 5°C and +35°C. However, the equipment 1 may also be used for controlling contaminants in gaseous flows having higher temperatures, such as for example combustion fumes, as better described hereinafter.

The equipment 1, constituting an additional element with respect to the conventional structure of a conveyed emission, is also suitable to be installed on previously existing plants.

Hereinafter, the term contaminants shall be used to indicate any substance harmful or damaging for the environment and/or for human and animal health contained in a gas flow to be treated at concentrations higher than those found in nature.

With particular reference to the attached figures, the equipment 1 for controlling contaminants in a gas flow (typically air) comprises a main duct 2 for transporting a main flow F of contaminated gas and means 3 for generating oxidising chemical species connected to the main duct 2 and adapted to introduce such oxidant species in the main gas flow F which flows within the latter.

According to the idea on which the present invention is based, the equipment 1 further comprises one or more by-pass circuits 4, each provided with at least one inlet opening 5 connected to a section 200 upstream of the main duct 2, and at least one outlet opening 6 connected to a section 201 downstream of the main duct 2. The by-pass circuit 4 is adapted to be traversed by a secondary gas flow F_{b} which is a fraction of the main flow F which flows within the main duct 2 extracted from the main duct 2 through the inlet opening 5 and re-introduced into the main duct 2 through the outlet opening 6, placed downstream of the inlet opening 5 with respect to the direction of advancement X of the main flow F of contaminated gas in the main duct 2. Therefore, the by-pass circuit 4 bypasses a portion 202 of the main duct 2 subtended between the upstream section 200 and the section 201 downstream of the main duct 2.

For this purpose, the equipment 1 comprises circulation means 7, adapted to make the secondary gas flow Fb flow from the inlet opening 5 to the outlet opening 6.

The by-pass circuit 4 may be equipped with more inlet openings 5 for extracting the secondary flow F_{b} at several points spaced along the main duct 2 and with several outlet openings 6 for re-introducing the secondary flow F_{b} at several points spaced along the main duct 2.

The means for generating oxidising chemical species are placed to intercept the by-pass circuit 4 and separate, in particular, in the latter a suction branch 17 and a delivery branch 18 with respect to the main duct 2. The means 3 for generating oxidising chemical species are adapted to introduce, into the secondary flow F_{b}, oxidising chemical species at a higher amount with respect to the amount required for obtaining an efficient oxidation of the contaminant chemical species contained in the latter. A first base amount of the oxidising chemical species introduced into the secondary flow F_{b} is adapted to react with the contaminant chemical species contained in the secondary flow F_{b} and a second excess amount of the oxidising chemical species, i.e. the part of the oxidising chemical species remaining after the latter reacts with the contaminants present in the secondary flow F_{b}, is adapted to be transported from the secondary flow F_{b} in the main flow F. The second excess amount of the oxidising chemical species introduced into the main flow F is thus capable of reacting with the contaminant substances present in the main flow F, oxidising them.

With the aim of facilitating a through mixing of the secondary gas flow F_{b} coming from the by-pass circuit 4 and containing the second excess amount of oxidising chemical species, with the main gas flow F, the equipment 1 comprises first mixing means 8 arranged in the main duct 2, downstream of the outlet opening 6.

Advantageously, the first mixing means 8 are of the passive type, not including mechanical stirring means or the like, and they are adapted to facilitate a close contact between the excess amount of oxidant species transported by the secondary flow F_{b} and the untreated portion of the gas flow F. For this purpose, the first mixing means 8 preferably comprise a mixing device equipped with a mesh structure and/or provided with regular through cavities, such as for example an alveolar type of structure. The device may for example be constituted by bodies superimposed with the meshes and/or the through cavities arranged staggered to create forced zig-zag paths for the gas, or it may be constituted by bundles of wires or nets or filling bodies contained in special baskets. The filling device is advantageously characterised by a large surface area and low loss of head and may for example be made of ceramic, glass, plastic or steel.

The equipment 1 according to the present invention is thus adapted to operate directly only on a fraction of global flow of contaminated gas to be treated which flows within the main duct 2, or only on the secondary flow F_{b} which flows in the by-pass circuit 4, generating oxidising chemical species therein. In addition, the equipment 1 operates indirectly on the remaining contaminated gas flow F, by means of the treated secondary gas flow F_{b} which is re-introduced into the main duct 2, in this secondary flow F_{b} there being present a concentration of residual oxidising chemical species capable of oxidising the contaminant chemical species present in the remaining untreated portion of the main flow F. The interaction of such residual oxidising chemical species with the contaminants present in the untreated portion of the main gas flow F is facilitated by a through mixing of the secondary flow F_{b} re-introduced into the main duct 2 with the main flow F flowing therein, allowed by the first mixing means 8.

Therefore, the equipment 1 according to the present invention is capable of reducing the contaminants present in high gas flow rates, with a low overall dimension, given that only one portion of the total gas flow intended to be treated is subjected to the direct action of the means for generating oxidising chemical species 3.

Advantageously, the flow rate of the secondary flow F_{b}, extracted from the main flow F and made to flow through the by-pass circuit 4, is comprised between 20% and 80%, and usually between 30% and 60%, of the flow rate of the main flow F.

The flow rate of the secondary gas flow F_{b} extracted from the main flow F and intended to be directly treated in the by-pass circuit 4 may vary as a function of the specific conditions of use of the equipment 1 and as a function of specific process choices that may be implemented from time to time. In particular, the flow rate of the secondary gas flow F_{b} may be determined, for example, as a function of the type and the concentration of contaminants present in the main gas flow F and as a function of the maximum concentration of contaminant substances tolerated in the main gas flow following the treatment F', or downstream of the first mixing means 8. Furthermore, the flow rate of the secondary flow F_{b} may for example be reduced by increasing the concentration of oxidising chemical species generated by the means for generating oxidising chemical species 3, and, thus, the concentration of oxidising excess chemical species with respect to the load of contaminants to be oxidised present in the secondary flow F_{b} which are introduced into the secondary flow F_{b} and thus into the main gas flow F. Thus, results comparable in terms of reduction of contaminants present in the main flow F of the gas to be treated may practically be obtained with different flow rates of the secondary flow F_{b}.

Advantageously, in the main duct 2, and preferably upstream of the section 200 upstream of the main duct 2, there may be arranged filtering means of the known type for example comprising, according to the application, filters of various types such as electrostatic filters, active carbon filters, metal filters, catalyst filters or other filters of the known type.

According to a preferred embodiment of the equipment 1 according to the present invention, the means for generating oxidising chemical species 3 comprise one or more capacitors 9, each of which is electrically connected to power supply means. The number of capacitors 9 provided for within the means for generating oxidising chemical species 3 depends on the condition of use of the equipment 1 and the specific process choices. Preferably, the means for generating oxidising chemical species 3 generally comprise a plurality of capacitors 9, as better outlined hereinafter.

The power supply means, per se known to a man skilled in the art and thus not described further in the present document, are adapted to electrically supply the capacitors 9, in particular with frequencies, waveforms and voltages determined as a function of the operating conditions of the equipment 1.

The secondary flow F_{b} preferably flows through an operating step of the means for generating oxidising chemical species 3 (for example the operating step is formed by one or more cylindrical containment tubes 11 as better described hereinafter) at contact with the capacitors 9, with turbulence, marked by a Reynolds number equal to at least 5000.

The turbulent flow facilitates generating oxidising chemical species in the secondary gas flow F_{b} by each capacitor 9 of the ionising area of each capacitor, i.e. the area within which each capacitor performs the ionising effect thereof, actually, affects the close surroundings of the capacitor and it is exhausted within a few millimetres from the external surface of the capacitor. The turbulent flow thus facilitates a greater exchange of the gas layer that hits the external surface of the capacitors, i.e. the gas layer that traverses the ionisation area, thus allowing generating a greater number of oxidising chemical species and improving the oxidation efficiency obtainable by using the equipment 1.

Preferably, each capacitor 9 comprises a pair of cylindrical armatures, including a first internal armature 12 and a second external armature 13 facing the first, separated by a dielectric, between which there is provided an alternating polarity for charging the armature having positive and negative sign alternatingly. Thus, each capacitor 9 has, around the external armature 13 thereof, an ionisation crown, i.e. an ionisation area shaped to form a crown which develops over the length of the capacitor 9.

Operatively, the capacitors 9 ionise the gas which flows at contact therewith, generating in the latter a plurality of ionised chemical species having oxidising properties. More in detail, the gas molecules subjected to the action of the capacitors 9 have an electrical axis that follows the force lines of the electric field generated by the capacitors 9, which are variable over time and in the space around the capacitors 9. This generates a vibration of the molecules, increasing the energy in the collisions between the molecules. The impacts between the molecules which are derived from this vibration generate a plurality of ionic species around the capacitor. At ambient temperature the energy exchanged in the collisions between the molecules is extremely low, and thus the probability of forming ionic species is low. In the oscillating electrical field produced by each capacitor 9 the gas molecules are oriented aligning along the lines of the field variable over time. Thus, the collisions due to the polarisation vibration of the molecules are more energetic and the formation of ionic species is more frequent. The ionic species thus formed, due to the high reactivity thereof, may recombine with each other and/or with other molecules present in the air forming molecule clusters. Though not in ionic form, many species formed in this manner reveal strong oxidising capacities. The energy for attaining these aggregations is derived both from heat vibration and from the impact between the molecular ions.

The oxidising chemical species generated by the capacitors 9 are thus mainly in molecular and cluster form.

Such oxidising chemical species have a sufficiently long life time which allow them to perform the oxidising actions thereof not only while intercepting the means for generating oxidising chemical species 3 and in the delivery branch 18 of the by-pass circuit 4, but also in the main duct 2, once conveyed from the secondary flow F_{b} into the latter and mixed with the main gas flow F by means of the first mixing means 8.

Generally the oxidising chemical species generated by the means for generating oxidising chemical species 3 in the secondary gas flow F_{b} have a life time comprised between 2 and 10 seconds. Advantageously, at least the 50% of the aforementioned oxidising chemical species have a life time of at least 5 seconds.

The length of the delivery branch 18 of the by-pass circuit 4 and the speed of the secondary flow F_{b} shall be such to allow the oxidising species generated by the means 3 to reach the main duct 2 within a time range considerably lower than the average life time of the oxidising species.

The means for generating oxidising chemical species 3 further preferably comprise one or more containment cylindrical tubes 11, each of which houses a capacitor 9 therein and it is arranged with the axis thereof parallel to the direction of advancement of the secondary flow F_{b} so as to be axially traversed by the latter. The gas which traverses the cylindrical contention tubes 11 is conveyed to flow into the interspace defined between each containment tube 11 and the external surface of the corresponding capacitor 9. Advantageously each capacitor 9 is held in position within the corresponding containment tube 11 by spacers 31.

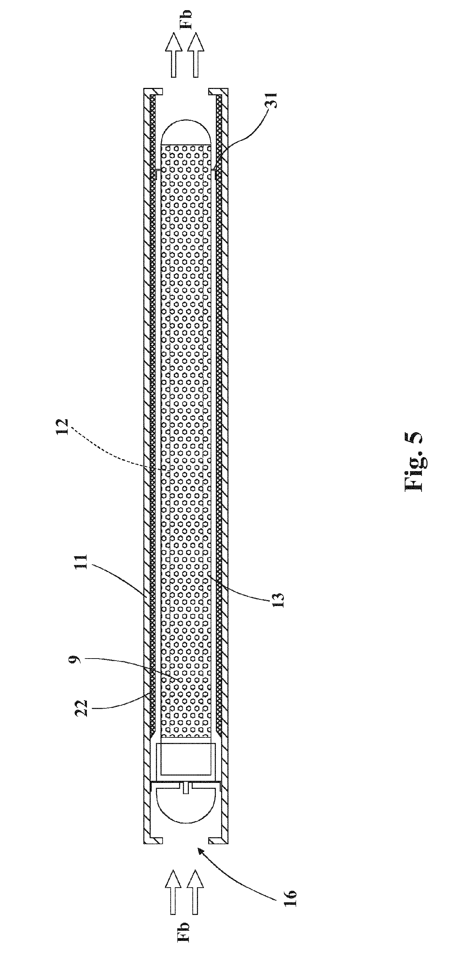

The containment tube 11 is advantageously shaped so as to facilitate the occurrence of said turbulence in the gas which hits the capacitor 9, as illustrated merely by way of example in figure 5.

Advantageously, the internal wall of the containment tube 11 may have roughness or channels or protrusions 22 obtained both by machining the material the containment tube 11 is made of, and by means of superimposition along the internal surface of one or more layers of ceramic, plastic or metal material. Furthermore, there may be provided a plurality of septa or fins adapted to facilitate the turbulence of the gas flow which traverses the containment tube 11 and/or adapted to force such gas flow to follow a forced path around the capacitor 9, for example with helical development.

In particular, the internal diameter of the containment tube 11 is preferably dimensioned so as to reduce the passage section of the gas, i.e. so as to determine the formation of an interspace for the passage of gas between the external perimeter wall of the capacitor 9 and the internal wall of the containment tube 11 in which the gas flow is strongly affected by the walls, thus leading to a high value of the Reynolds number. Thus, a higher amount of gas which traverses the containment tube 11 is forced to traverse the ionisation crown defined around each capacitor 9.

Due to such solutions, the means for generating oxidising chemical species 3 are capable of generating in the secondary gas flow F_{b} a greater amount of oxidising chemical species, thus improving the oxidising efficiency of the equipment 1.

These advantageous characteristics of the means for generating oxidising chemical species 3 described above, i.e. the capacity of increasing the concentration of oxidising chemical species generated by the means 3 due to the creation of a flow with turbulence which hits the capacitors, in particular by means of the housing of each capacitor in a corresponding containment tube of suitable diameter and/or suitably shaped, can per se be exploited in the use of means 3 in any duct or chamber and for the treatment of any fluid, whether a gas or a liquid, containing or not containing contaminant chemical species.

As mentioned previously, the means for generating oxidising chemical species 3 preferably comprise a plurality of capacitors 9, which are arranged within the respective containment tubes 11 placed one adjacent to the other and operate in parallel, so that the secondary gas flow F_{b} is distributed between the containment tubes 11 and axially flows into the latter, at contact with the capacitors 9. Therefore, the gas which exits from each containment tube 11 and which was made oxidant by the capacitor 9 contained therein does not flow through a second containment tube 11, but it is directly transported towards the main duct 2 through the delivery branch 18 of the by-pass circuit 4.

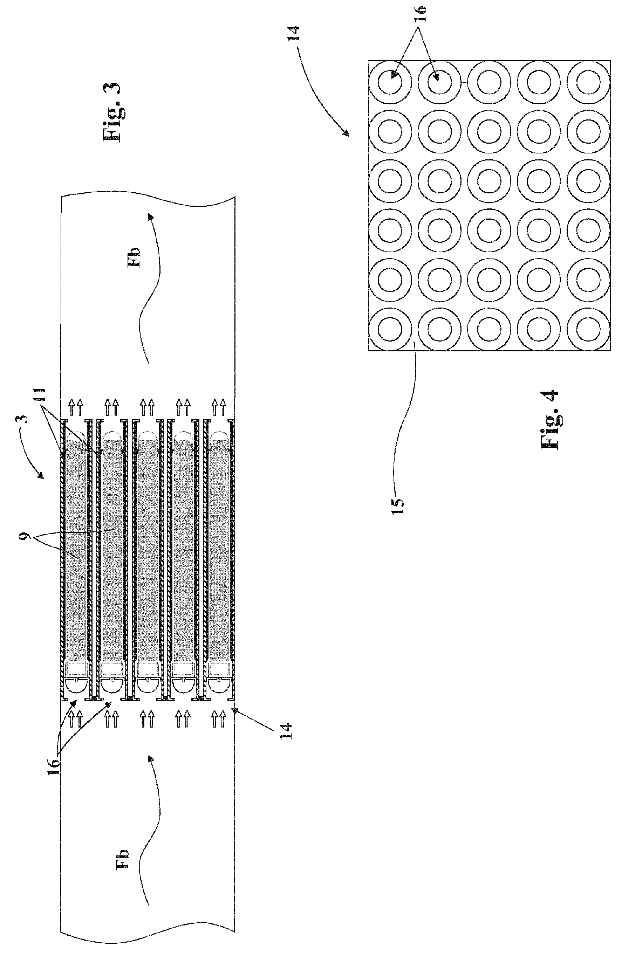

The means for generating oxidising chemical species 3 comprise, in particular, a support structure 14 on which there are mounted the containment tubes 11 and the capacitors 9, each of the latter housed within a corresponding containment tube 11. The support structure 14 may for example comprise a base 15 on which there are obtained a plurality of through holes 16 each communicating with a respective containment tube 11, as illustrated purely by way of example in Figures 3 and 4, so that the secondary gas flow F_{b} which flows through the suction branch 17 of the by-pass circuit 4 is forced through the through holes 16 of the base 15 of the support structure 14 of the means 3, towards the internal of the containment tubes 11 containing the capacitors 9.

The support structure 14 of the means 3 may have a different shape and construction characteristics with respect to those described briefly, as long as adapted to convey the secondary gas flow F_{b} towards the internal of the containment tubes 11 to be supported.

With the aim of facilitating the most homogeneous distribution of the oxidising chemical species generated by the means 3 in the secondary gas flow F_{b} which is transported through the delivery branch 18 towards the main duct 2, the means for generating oxidising chemical species 3 advantageously comprise second mixing means 10, placed downstream of the containment tubes 11, in order to mix the gas containing oxidising chemical species exiting from the latter.

In case of malfunctioning of one of the capacitors 9, hence the latter not be capable of generating oxidising chemical species in the gas which traverses the containment tube 11 in which it is housed, the second mixing means 10 facilitate the mixing of the gas which exits from all containment tubes 11, thus preventing the creation of gas portions without oxidising chemical species in the treated secondary gas flow F_{b}.

Besides the capacitors 9, the means for generating oxidising chemical species 3 may also comprise further means for producing oxidising chemical species, such as for example UV sources and/or catalytic converters and/or ozone generators, exploiting the same principle of generating an excess amount of oxidising species with respect to that required for the oxidation of the contaminants present in the secondary gas flow F_{b}.

Advantageously, the equipment 1 may comprise means 23 for injecting an auxiliary gas flow, preferably oxygen, connected to the suction branch 17 of the by-pass circuit 4, which are adapted to insufflate controlled quantities of auxiliary gas into the suction branch 17. Preferably, the injection means 23 comprise a valve 24 for allowing, interrupting and adjusting the supply of auxiliary gas into the suction branch 17.

The increase of the flow rate of the gas flow which traverses the means for generating oxidising chemical species 3 and, in particular, the increase of the concentration of oxygen present in such gas flow is actually capable of increasing the production efficiency of oxidising species in the gas flow.

Advantageously, the equipment 1 according to the present invention further comprises a first sampling opening 19 obtained in the suction branch 17 of the by-pass circuit 4 and a second sampling opening 20 obtained in the delivery branch 18 of the by-pass circuit. Preferably, the equipment 1 further comprises at least one third sampling opening 21 obtained in the main duct 2, downstream of the first mixing means 8.

The equipment 1 further comprises detection means (not illustrated) adapted to be introduced into the suction branch 17 and/or into the delivery branch 18, respectively through the first and/or the second sampling opening to detect the chemical/physical characteristics of the secondary flow F_{b} upstream and/or downstream of the means for generating oxidising chemical species 3, or adapted to be introduced into the main duct through the third sampling opening 21 to detect the chemical/physical characteristics of the treated main flow F', downstream of the first mixing means 8. In particular, the detection means comprise sensors of the specific and/or non-specific type adapted to detect the concentration of contaminant chemical species present in the secondary flow F_{b} upstream and/or downstream of the means for generating oxidising chemical species 3, and in the treated main flow F', downstream of the first mixing means 8.

The detection means may also advantageously comprise probes for measuring the temperature or humidity and instruments for measuring the pressure and the speed of the gas flow at the sampling points.

The detection means allow monitoring the operation of the equipment 1 over time.

Advantageously, the equipment 1 comprises means 25 for controlling the temperature of the secondary flow F_{b}, which are connected to the suction branch 17 of the by-pass circuit 4. The means 25 for controlling the temperature for example comprise a heat exchanger and they are adapted to heat or cool the secondary gas flow F_{b}, depending on the temperature detected by the detection means at the sampling points upstream and downstream of the means for generating oxidising chemical species 3, so as to allow the ideal operating conditions for the means 3. The physical characteristics of the gas flow which traverses the means for generating oxidising chemical species 3, such as in particular the temperature and humidity, actually affect the operation of the means for generating oxidising chemical species 3, or their capacity to generate oxidising species in the gaseous flow that traverses them. Preferably, the temperature of the secondary gas flow F_{b} is comprised between -5°C and 35°C as mentioned previously.

More in detail, should the equipment 1 be used for controlling contaminants in a hot gas flow, particularly having a temperature exceeding 35°C, such as for example combustion fumes, the means 25 for controlling the temperature are adapted to cool the secondary gas flow F_{b}, before being made to pass in the latter through the means for generating oxidising chemical species 3.

The equipment 1 further comprises humidity controlling means 26 placed upstream of the means for generating oxidising chemical species 3 and, preferably, arranged in the suction branch 17. Advantageously, the humidity controlling means 26 comprise an atomiser adapted to be actuated when the humidity values detected by the detection means in the sampling points upstream and/or downstream of the means for generating oxidising chemical species 3, drop below a preset threshold value, preferably equal to a relative humidity value of about 70%. In case of gaseous flows characterised by an excess amount of humidity, the humidity controlling means 26 comprise a heat exchanger for removing the excess humidity through condensation.

The circulation means 7 for example comprise a fan. Preferably the circulation means 7 are arranged to intercept the suction branch 17, for suctioning the secondary gas flow F_{b} into the by-pass circuit 4.

The means 23 for injecting an auxiliary gas flow, the means 25 for controlling the temperature, the means 26 for controlling humidity and the first sampling opening 19 may be arranged each in the suction branch 17 of the by-pass circuit both upstream or downstream of the circulation means 7.

According to a preferred embodiment thereof, the equipment 1 according to the present invention comprises a control unit (not illustrated) at least connected to the detection means, to the circulation means 7 and to the power supply means of the capacitors 9, which is adapted to adjust the flow rate of the secondary flow F_{b} suctioned in the by-pass circuit 4 by the circulation means 7 and/or an electrical variable of the power supply means (such as in particular the frequency and/or the waveform and/or the voltage) which supply the capacitor 9, as a function of the concentration of contaminant chemical species detected by the detection means in particular in the secondary flow F_{b} upstream and downstream of the means for generating oxidising chemical species 3. Advantageously the control unit comprises a programmable logic controller and it is adapted to receive the signals coming from the detection means and thus control the operation of the power supply means of the capacitors 9 and the circulation means 7, with the aim of guaranteeing the ideal operation of the means for generating oxidising chemical species 3 and, thus, generating an amount of oxidising species in the secondary gas flow F_{b} sufficient to reduce the contaminant chemical species present in the secondary flow F_{b} and in the main flow F.

Preferably, the control unit is also connected to the means 23 for injecting an auxiliary gas flow, to the means 25 for controlling the temperature and the humidity controlling means 26 and thus adjusts the amount of auxiliary gas possibly introduced into the by-pass circuit 4, as well as the temperature and the humidity of the secondary gas flow F_{b} as a function of the chemical/physical characteristics of the secondary gas flow F_{b} of interest detected by the detection means.

Advantageously the equipment 1 may also comprise one or more filters, arranged upstream of the means for generating oxidising chemical species 3 for the removal of the particulate of greater dimensions possibly present in the main flow F or in the secondary gas flow F_{b}.

A process for controlling contaminants in a gas flow, in particular by means of an equipment of the type described above, regarding which the same reference numbers shall be maintained hereinafter, also forms an object of the present invention. Such process comprises the operating steps described below.

There is initially provided for a step for extracting a secondary gas flow F_{b} from a section 200 upstream of a main gas flow F containing contaminant chemical species. In particular, the extracted secondary gas flow F_{b}, which is a fraction of the main gas flow F, is preferably made to flow through a by-pass circuit 4.

The process thus comprises a step for generating oxidising chemical species in the secondary gas flow F_{b}. In particular, such oxidising chemical species are generated in the secondary gas flow F_{b} in greater amounts with respect to the amount required in order to oxidise the contaminant chemical species contained in in the latter. In a first reduction step, the contaminant chemical species (or at least a part of the contaminant chemical species) present in the secondary gas flow F_{b} are oxidised by means of a first base amount of the oxidising chemical species generated in the aforementioned generation step.

Then, there is provided a step for re-introducing in a section 201 downstream of the main flow F of the secondary gas flow F_{b} containing a second excess amount of oxidising chemical species and a step for closely mixing the secondary gas flow F_{b} with the main flow F of contaminated gas.

In a second reduction step, the contaminant chemical species (or at least a part of the contaminant chemical species) present in the main flow F, which was mixed with the secondary flow F_{b}, are oxidised by means of the second excess amount of oxidising chemical species transported by the secondary flow F_{b}.

Upon activating the means for generating oxidising chemical species 3, the steps described above are continuosly carried out the one after the other.

The process according to the present invention allows rapidly, safely and efficiently reducing the contaminant chemical species present in a gaseous flow generating oxidising chemical species in just one portion of such gaseous flow. The latter are capable of immediately oxidising the contaminant chemical species present in the portion of the gaseous flow in which they were generated and, subsequently, the contaminant chemical species present in the remaining gaseous flow, once the portion of the gaseous flow containing the oxidising chemical species is recombined with the latter.

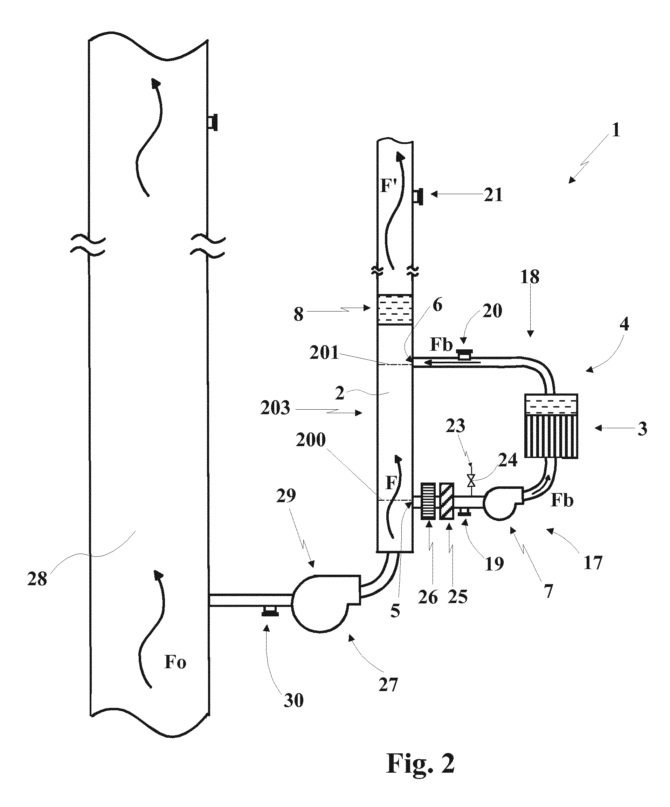

The equipment 1 according to the present invention, advantageously realized in small dimensions, is also suitable to be used in a method for calibrating the equipment. In particular, the equipment 1 is used in such case for evaluating the operating parameters of the equipment 1 in scale when used to reduce the contaminants present in a known flow of the gas F₀ to be treated. In this case, the main duct 2 of the equipment 1 is used as sample duct, adapted to reproduce the gas flow to be treated Fo in lower scale as illustrated in Figure 2.

This allows determining the ideal operating parameters of the equipment 1 required to reduce the contaminants present in the gas flow to be treated F₀ and obtain a clean gas flow having a minimum concentration of contaminant chemical species. In particular, this allows determining the flow rate of the secondary flow F_{b} which should be extracted from the main flow F, the number of capacitors 9 of the means for generating oxidising chemical species 3 as well as the frequencies, waveforms and voltages to be applied thereto, the temperature and humidity conditions of the secondary gas flow F_{b}, and the suitability or unsuitability of injecting an auxiliary gas flow into the by-pass circuit 4 and the flow rate of such possible auxiliary flow.

Thus, this allows accurately calibrating the equipment 1 and verifying the operation thereof and the reduction efficiency before the installation of an equipment in suitable scale in loco, for example in a chimney of an industrial plant. For example, this also allows calibrating the equipment 1 in a laboratory or however in a different place with respect to the definite site of use of the equipment and monitoring the operation thereof over the desired period of time.

For this purpose, the equipment 1 comprises a drawing branch 27 which connects the main duct 2 to a source of a gas flow F₀ to be treated, such as for example a primary piping 28 within which there flows the gas flow F₀ to be treated, to receive the main gas flow F from the latter. In the latter there is reproduced the chemical/physical and fluid-dynamic characteristics of the gas flow F₀ to be treated, in scale, having in particular the same speed, obtained by selecting the flow rate F as a function of the diameter of the main duct 2, and the same concentration of contaminant chemical species of the gas flow F₀ to be treated. Thus, this configuration of the equipment 1, allows determining, in scale, the operating parameters of the equipment 1 to reduce the desired amount of contaminant chemical species present in the gas flow F₀ to be treated.

Preferably, on the drawing branch 27 there are provided suctioning means 29, such as for example a fan for drawing, with the predefined flow rate, the main gas flow F from the source of the gas flow F₀ to be treated. The suctioning means 29 may however also be provided downstream of the main duct 2, or only provided on the suction branch 17 of the by-pass circuit, to create a vacuum adapted to attract the main gas flow F in the drawing branch 27 and in the main duct 2. On the drawing branch 27 there is also advantageously provided a fourth sampling opening 30, through which the detection means may be introduced into the drawing branch 27 to detect the chemical/physical and/or fluid-dynamic characteristics of the gas flow in the latter.

Advantageously the detection means of the equipment 1, when used for calibrating the equipment, comprise two or more non-specific sensors, which allow detecting the concentration of the contaminant chemical species present upstream and downstream of the means for generating oxidising chemical species 3 and possibly in the main duct 2 downstream of first mixing means 8 and in the drawing branch 27.

A method for calibrating the operating parameters of an equipment for controlling contaminants in a gas flow adapted for reducing the contents of contaminant chemical species in a gas flow F₀ to be treated to obtain a cleaned gas flow also forms an object of the present invention. Such method comprises a step for defining a concentration threshold value for the contaminant chemical species in the clean gas flow, or defining a maximum concentration value of contaminant chemical species tolerated in the gas flow after being treated in the latter. The method further comprises a calibration step, during which the operating parameters of the equipment 1 are determined in scale, as better described hereinafter, and a step for proportioning the operating parameters dependent on the flow rate of the flow to be treated as a function of the flow rate of the gas flow Fo to be treated.

More in detail, the calibration step comprises a first step for extracting a main gas flow F from the gas flow F₀ to be treated, containing the aforementioned contaminant chemical species and a second step for extracting a secondary gas flow F_{b} from a section 200 upstream of the main gas flow F. During the calibration step, the main gas flow F is not necessarily extracted from a primary piping 28 within which the gas flow F₀ to be treated flows, as illustrated in Figure 2. The gas flow F₀ to be treated could actually be reproduced also by continuous injection or evaporation of one or more volatile organic or inorganic compounds or odoriferous substances constituting the contaminated chemical species known to be present in the gas flow F₀ to be treated and intended to be reduced. Thus, the main gas flow F constitutes a reproduction of the gas flow F₀ to be treated, having the same chemical and fluid-dynamic characteristics of the latter.

Thus, the calibrating step comprises a step for generating oxidising chemical species in the secondary gas flow F_{b} extracted from the main gas flow F and a first step for reducing the contaminant chemical species present in the secondary gas flow F_{b} by a first base amount of the oxidising chemical species. Furthermore, there is provided for a step for re-introducing the secondary gas flow F_{b}, containing a second excess amount of the oxidising chemical species, into a section 201' downstream of the main gas flow F and one or more steps for the through mixing of the secondary gas flow F_{b} with the main contaminated gas flow F.

In a second reduction step, the contaminant chemical species present in the main flow F mixed with the secondary flow F_{b} are oxidised by means of the second excess amount of the oxidising chemical species.

The calibrating step further comprises one or more steps for detecting the concentration of the contaminant chemical species in the main treated gas flow F', i.e. previously mixed with the secondary flow F_{b}, downstream of the point for reintroducing the latter into the main gas flow F, so as to verify the capacity of the equipment 1 to reduce contaminant chemical species. Thus, there is provided for a step for adjusting the amount of oxidising chemical species generated in the secondary flow F_{b}, advantageously obtained by means of the control unit, and a step for identifying a minimum amount of oxidising chemical species that are to be generated in the secondary flow F_{b} so that the concentration of the contaminant chemical species detected in the main gas flow F is lower than the predefined threshold value. Depending on the minimum amount of oxidising chemical species identified, in a calibration step, the operating parameters of the equipment may be defined in order to generate the aforementioned minimum amount of oxidising chemical species, or in particular the number of capacitors 9 to be provided with the means for generating oxidising chemical species 3, and the frequencies, waveforms and voltages to be applied thereto, the temperature and humidity conditions of the secondary gas flow F_{b}, and the suitability or unsuitability of injecting an auxiliary gas flow into the by-pass circuit 4 and the flow rate of such possible auxiliary flow.

Upon completing the calibrating step described above, there follows the step of proportioning the operating parameters dependent on the flow rate of the flow to be treated mentioned beforehand, in which the parameters defined through the calibrating step are compared to the actual flow rate value of the gas flow F₀ to be treated.

Therefore, the method thus conceived attains the preset objects.

However, in the practical implementation thereof, it may also take shapes and configurations different from the one illustrated above without departing from the present scope of protection. Furthermore, the details may be replaced by other technically equivalent elements and the dimensions, shapes and materials used may vary according to the technical requirements.

## Claims

1. Equipment for controlling contaminants in a gas flow, comprising a main duct (2) for transporting a main flow (F) of contaminated gas in a direction of advancement (X);
and **characterised in that** it further comprises:
- at least one by-pass circuit (4) equipped with at least one inlet opening (5) connected to a section (200) upstream of said main duct (2), and at least one outlet opening (6) connected to a section (201) downstream of said main duct (2) for the extraction of a secondary gas flow (Fb) from said main duct (2) through said inlet opening (5) and the re-introduction of said secondary gas flow (Fb) into said main duct (2) through said outlet opening (6) by-passing a portion (202) of said main duct (2) subtended between the upstream section (200) and the downstream section (201) of said main duct (2) taken with respect to said direction of advancement (X) of said main flow (F);
- circulation means (7) for making said secondary gas flow (Fb) flow from said inlet opening (5) to said outlet opening (6);
- means for generating oxidising chemical species (3) arranged to intercept said at least one by-pass circuit (4) and adapted to generate oxidising chemical species in said secondary flow (Fb), a first base amount of said oxidising chemical species being adapted to react with the contaminant chemical species contained in said secondary flow (F_{b}) in order to oxidise them and a second excess amount of said oxidising chemical species being adapted to be transported by said secondary flow (F_{b}) into said main flow (F) in order to oxidise the contaminant chemical species contained in the latter;
- first mixing means (8) arranged in said main duct (2) downstream of said outlet opening (6) in order to mix said secondary flow (F_{b}), containing said second excess amount of said oxidising chemical species, with said main flow (F).

2. Equipment for controlling contaminants in a gas flow according to claim 1, **characterised in that** said means for generating oxidising chemical species (3) comprise at least one capacitor (9) electrically connected to power supply means and arranged in an operating portion of said by-pass circuit (4) susceptible to conduct said secondary flow (F_{b}) to hit said at least one capacitor (9) with turbulence, **characterised by** a Reynolds number equal to at least 5000.

3. Equipment for controlling contaminants in a gas flow according to claim 2, **characterised in that** said at least one capacitor (9) comprises a pair of cylindrical armatures, including a first internal (12) and a second external (13) facing the first, separated by a dielectric between which a voltage with alternating polarities is obtained for charging said armatures with alternating positive and negative signs and said means for generating oxidising chemical species (3) comprise at least one cylindrical containment tube (11) housing said capacitor (9) therein and arranged with the axis thereof parallel to the direction of advancement of said secondary flow (F_{b}) so as to be axially traversed by said secondary flow (F_{b}), the gas intercepting said means for generating oxidising chemical species (3) being conveyed to flow into the interspace defined between said at least one containment tube (11) and the corresponding capacitor (9) and said containment tube (11) having an internal diameter and/or being shaped in order to generate said turbulence of said secondary flow (F_{b}).

4. Equipment for controlling contaminants in a gas flow according to claim 1, **characterised in that** said means for generating oxidising chemical species (3) separate in said by-pass circuit (4) a suction branch (17) and a delivery branch (18).

5. Equipment for controlling contaminants in a gas flow according to claim 4, **characterised in that** it comprises at least one sampling opening (19) obtained in said main duct (2) or in said by-pass circuit (4), and detection means introduced in said sampling opening (19) to detect the chemical/physical characteristics of said secondary flow (F_{b}), and in particular the concentration of contaminant chemical species present therein.

6. Equipment for controlling contaminants in a gas flow according to claim 4, **characterised in that** it comprises means (23) for injecting an auxiliary gas flow connected to the suction branch (17) of said by-pass circuit (4).

7. Equipment for controlling contaminants in a gas flow according to claim 4, **characterised in that** it comprises means (25) for controlling the temperature of said secondary flow (F_{b}), connected to the suction branch (17) of said by-pass circuit (4).

8. Equipment for controlling contaminants in a gas flow according to any one of the preceding claims, **characterised in that** it comprises humidity controlling means (26) arranged upstream of said means for generating oxidising chemical species (3).

9. Equipment for controlling contaminants in a gas flow according to claims 2 and 5, **characterised in that** it comprises a control unit connected at least to said detection means, to said circulation means (7) and to said power supply means of said at least one capacitor (9), and adapted to adjust at least the flow rate of said secondary flow (F_{b}) suctioned in said by-pass circuit (4) by said circulation means (7) and/or an electrical variable of said power supply means to said at least one capacitor (9), as a function of the concentration of contaminant chemical species detected by said detection means.

10. Equipment for controlling contaminants in a gas flow according to any one of the preceding claims, **characterised in that** it comprises a drawing branch (27) which connects said main duct (2) to a source of a gas flow to be treated (F₀), to receive from the latter, said main gas flow (F) having the same chemical/physical and fluid-dynamic characteristics of said gas flow to be treated (Fₒ), with the aim of determining, in scale, the operating parameters of said equipment (1) to at least partly reduce the contaminant chemical species present in said gas flow to be treated (F₀) and obtain a cleaned gas flow having a predetermined maximum concentration of contaminant chemical species.

11. Equipment for controlling contaminants in a gas flow according to claims 5 and 10, **characterised in that** said detection means comprise at least one non-specific sensor.

12. Process for controlling contaminants in a gas flow, **characterised in that** it comprises the following operative steps:
- a step for extracting a secondary gas flow (F_{b}) from a section (200) upstream of a main gas flow (F) containing contaminant chemical species;
- a step for generating oxidising chemical species in said secondary gas flow (F_{b});
- a first step for reducing the contaminant chemical species present in said secondary gas flow (F_{b}) by means of a first base amount of said oxidising chemical species;
- a step for re-introducing said secondary gas flow (F_{b}) containing a second excess amount of said oxidising chemical species in a section (201) downstream of said main flow (F);
- at least one step for throughly mixing said secondary gas flow (F_{b}) with said main flow (F) of contaminated gas;
- a second step for reducing the contaminant chemical species present in said main flow (F) mixed with said secondary flow (F_{b}) by means of said second excess amount of said oxidising chemical species.

13. Method for calibrating the operating parameters of an equipment for controlling contaminants in a gas flow adapted to reduce the contents of contaminant chemical species in a gas flow (F₀) to be treated for obtaining a cleaned gas flow, **characterised in that** it comprises:
- a step for defining a concentration threshold value for the contaminant chemical species in a cleaned gas flow;
- a calibration step comprising:
- a first step for extracting a main gas flow (F) from a section (200) upstream of a gas flow (F₀) to be treated containing said contaminant chemical species;
- a second step for extracting a secondary gas flow (F_{b}) from said main gas flow (F);
- a step for generating oxidising chemical species in said secondary gas flow (Fb) extracted from said main gas flow (F);
- a first step for reducing the contaminant chemical species present in said secondary gas flow (F_{b}) by means of a first base amount of said oxidising chemical species;
- a step for re-introducing said secondary gas flow (F_{b}), containing a second excess amount of said oxidising chemical species, in a section (201) downstream of said main gas flow (F);
- at least one step for throughly mixing said secondary gas flow (F_{b}) with said main contaminated gas flow (F);
- a second step for reducing the contaminant chemical species present in said main flow (F) mixed with said secondary flow (F_{b}) by means of said second excess amount of said oxidising chemical species;
- at least one step for detecting the concentration of said contaminant chemical species in said main gas flow, previously mixed with said secondary gas flow (F_{b});
- a step for adjusting the amount of oxidising chemical species generated in said secondary flow (F_{b});
- a step for identifying a minimum amount of oxidising chemical species generated in said secondary flow (F_{b}) so that the concentration of said contaminant chemical species detected in said main gas flow in said detection step is lower than said predefined threshold value;
- a step for calibrating the operating parameters of said equipment 1 in order to generate said minimum amount of oxidising chemical species;
- a step for proportioning said operating parameters dependent on the flow rate of said gas flow (F₀) to be treated, as a function of the flow rate of said gas flow (F₀) to be treated.
